# EUROPEAN PATENT SPECIFICATION

(11) **EP 1 443 035 B1**
(45) Date of publication and mention of the grant of the patent: **11.04.2012**
(21) Application number: 01976782.1
(22) Date of filing: 19.10.2001
(51) Int. Cl.: C07C 11/167, C07C 11/18, C07C 7/05, C07C 7/20

(54) **PROCESS AND APPARATUS FOR SEPARATION AND PURIFICATION OF CONJUGATED DIENE**
VERFAHREN UND VORRICHTUNG ZUR TRENNUNG UND AUFREINIGUNG VON KONJUGIERTEM DIEN
PROCEDE ET DISPOSITIF DE SEPARATION ET DE PURIFICATION DE DIENE CONJUGUE

(43) Date of publication of application: 04.08.2004
(73) Proprietor: Zeon Corporation, Chiyoda-ku Tokyo 100-8246 (JP)
(72) Inventor: KANAUCHI, Masanobu, c/o ZEON CORPORATION, Tokyo 100-8323 (JP); KAJI, Masayoshi, MIZUSHIMA PLANT ZEON CORPORATION, Kurashiki-shi, Okayama 711-0934 (JP)
(74) Representative: Vossius & Partner
(86) International application number: PCT/JP2001/009208
(87) International publication number: WO 2003/035588

(56) References cited:
- JP-A- 61 130 242
- US-A- 4 859 286
- DATABASE WPI Week 199848 Thomson Scientific, London, GB; AN 1998-563463 XP002548853 & JP 10 251662 A (JAPANESE GEON CO LTD) 22 September 1998 (1998-09-22)

## Description

### TECHNICAL FIELD

The present invention relates to a method and apparatus for separating and purifying conjugated diene capable of efficiently suppressing generation of popcorn polymer inside a purification apparatus when separating and purifying high purity isoprene, butadiene and other conjugated diene from petroleum distillates.

### BACKGROUND ART

Conjugated diene, such as 1,3-butadiene, isoprene and chloroprene as unsaturated hydrocarbons, are liable to accidentally generate porous insoluble polymer, known as popcorn polymer, both in liquid and gas phases. Particularly, industrial distillation satisfies various conditions liable to generate the popcorn polymer, such as suitable temperature at operation, high purity monomer, coexistence of gas and liquid phases, moisture ingredient, and existence of iron rust. Once this popcorn polymer has been generated, it is made to a core and the popcorn polymer exponentially increases around the core leading to rapid blockage in an apparatus. Also, this polymer is strong that it is insoluble in all known solvents and does not melt even if heated. In order to remove this polymer, there is no other suitable process but cleaning by mechanical means. This cleaning requires to suspend the apparatus for a time and so that it is not possible to avoid the economical disadvantage.

Moreover, the mechanical cleaning cannot completely remove the polymer that when the operation begins, a small amount of polymer that was left behind is made to the core and the polymer increases again. As a method of preventing polymerization of conjugated diene inside a separation and purification apparatus, a variety of proposals have been made. For example, the Japanese Unexamined Patent Publication No. 50-112304 discloses a method of distilling C5 hydrocarbon in the presence of a di-lower alkylhydroxylamine and it describes that the method allows the suppression of generation of a popcorn polymer at distillation of isoprene.

Also, the Japanese Unexamined Patent Publication No. 56-81526 discloses a method of extractive distillation in the presence of furfural and a furfural condensation product in an extracting solvent. Furthermore, the Japanese Examined Patent Publication No.45-19682 discloses a method of extractive distillation for conjugated diene hydrocarbon by adding a polymerization inhibitor or a polymerization chain transfer agent in an extracting solvent. In here, it discloses that adding a polymerization inhibitor or a polymerization chain transfer agent in an extracting solvent is also possible.

JP 10251662 A discloses a process for separating conjugated dienes by extractive distillation of a petroleum fraction containing conjugated dienes with an extractive distillation tower, the oxygen concentration in the fraction and in the extractant are measured; the weight of oxygen inflow is calculated from the measured oxygen concentrations and the amounts of the fraction and extractant supplied; and the amount of a polymerisation inhibitor supplied to the tower is controlled in proportion to the oxygen inflow weight.

However, according to the method described in these publications, a more than necessary amount of a polymerization inhibitor was added to suppress generation of popcorn polymer inside the apparatus in some cases, consequently, costs of separation and purification tended to rise.

### DISCLOSURE OF THE INVENTION

An object of the present invention is to provide a method and apparatus for separating and purifying conjugated diene, capable of efficiently and stably suppressing generation of popcorn polymer in the apparatus.

According to the present invention, a method of separating and purifying conjugated diene comprises the steps of
obtaining a first bottom product by distilling a hydrocarbon mixture including 80% or more conjugated diene under an environment in the presence of a polymerization inhibitor in a lower boiling distillation column;
obtaining a second bottom product by distilling said first bottom product under an environment in the presence of the polymerization inhibitor in a higher boiling distillation column;
measuring each of concentrations of the polymerization inhibitors included in said first bottom product and second bottom product; and
controlling the concentration of the polymerization inhibitor included in said first bottom product and/or second bottom product by changing a supply amount of the polymerization inhibitor to said lower boiling distillation column and/or higher boiling distillation column in accordance with the measured concentration of the polymerization inhibitor.

To attain the above object, an apparatus for separating and purifying conjugated diene which is used in the present invention comprises

a lower boiling distillation column for obtaining a first bottom product by distilling a hydrocarbon mixture including 80% or more conjugated diene under an environment in the presence of a polymerization inhibitor;
a higher boiling distillation column for obtaining a second bottom product by distilling the first bottom product under an environment in the presence of a polymerization inhibitor;
a measurement means for measuring each of concentrations of the polymerization inhibitors included in the first bottom product and second bottom product respectively;
a supply system for supplying a polymerization inhibitor newly to the lower boiling distillation column and/or higher boiling distillation column; and
a control means for controlling the concentration of the polymerization inhibitor included in the first bottom product and/or second bottom product by changing the supply amount of the polymerization inhibitor to the lower boiling distillation column and/or higher boiling distillation column in accordance with the measured concentration of the polymerization inhibitor.

In the present invention, it is preferable to use an analyzing apparatus, such as an ion chromatograph, a liquid chromatograph and a gas chromatograph, for measuring concentration of the polymerization inhibitor.

In the present invention, as a method of changing a supply amount of a polymerization inhibitor to a distillation column, for example, when the measured concentration of the polymerization inhibitor exceeds the upper limit value of a reference range, the supply amount of the polymerization inhibitor to the distillation column may be decreased, while when the measured concentration of the polymerization inhibitor is below the lower limit value of the reference range, the supply amount of the polymerization inhibitor to the distillation column may be increased. In the present invention, a means to change the supply amount of the polymerization inhibitor to the distillation column may be manually controlled or automatically controlled.

In the present invention, as a method of changing a supplying stage of a polymerization inhibitor to a distillation column, for example, when the measured concentration of the polymerization inhibitor exceeds the upper limit value of a reference range, the supplying stage of the polymerization inhibitor to the distillation column may be lowered, while when the measured concentration of the polymerization inhibitor is below the lower limit value of the reference range, the supplying stage of the polymerization inhibitor to the distillation column may be raised. In the present invention, a means to change the supplying stage of the polymerization inhibitor to the distillation column may be manually controlled or automatically controlled.

A hydrocarbon mixture including conjugated diene, which can be applied to the present invention is normally a hydrocarbon mixture including conjugated diene of C4 or more obtained by cracking naphtha and separating ethylene, propylene and other C2 and C3 hydrocarbons, preferably a C4 hydrocarbon fraction or C5 hydrocarbon fraction, more preferably a C4 hydrocarbon fraction including butadiene or a C5 hydrocarbon fraction including isoprene.

In a hydrocarbon mixture-including conjugated diene, which can be applied to the present invention, the content(concentration) of conjugated diene is previously heightened by extractive distillation or so. In this case, conjugated diene included in the hydrocarbon mixture is normally, 80% or more, preferably, 90% or more, and more preferably, 95% or more.

A polymerization inhibitor, which can be used in the present invention, is used for suppressing generation of popcorn polymer inside the apparatus and not particularly limited.

Concretely, as to inhibit and suppress polymerization by scavenging radicals with stable radicals, 1,1-diphenyl-2-picrylhydrazyl, 1,3,5-triphenyl ferudazyl, 2,6-di-t-butyl-α-(3,5-di-t-butyl-4-oxo-2,5-cyclohexanediene-1-indene)-p-tolyloxy, 2,2,6,6-tetramethyl-4-piperidone-1-oxyl, N-(3-N-oxyanilino-1,3-dimethylbutylidene)-aniline oxide, 2-(2-cyanopropyl)-ferudazyl; as to inhibit and suppress polymerization by chain transfer reaction, ones having activated NH-bond such as diphenylpicrylhydrazine, diphenylamine, diethylhydroxylamine, dimethylhydroxylamine, methylethylhydroxylamine, dipropylhydroxylamine, dibutyhydroxylamine, dipentylhydroxylamine; ones having OH-bond of phenol such as hydroquinone and t-butylcatechol; ditiobenzoyldisulfide, p,p'-ditolyl trisulfide, p,p'-ditolyl tetrasulfide, dibenzyl tetrasulfide, tetraethylthiuram disulfide; as to inhibit and suppress polymerization by addition reaction, oxygen, sulfur, anthracene, 1,2-benzanthracene, tetracene; benzoquinone derivatives such as chloranil, p-benzoquinone, 2,6-dichlorbenzoquinone, 2,5-dichlor benzoquinone, nitro compounds such as furfurylidene-malononitrile and m-dinitrobenzene, nitroso compounds such as nitrosobenzene, 2-methyl-2-nitrosopropane; further, metal salts such as ferric chloride and ferric bromide are exemplified.

Out of the above polymerization inhibitors, ones that inhibit and suppress the polymerization by chain transfer reaction, particularly di-lower aklylhydroxylamine is preferable. Concretely, diethylhydroxylamine is preferable.

Further, these polymerization inhibitors may be used alone or in combination of two or more. An amount of the polymerization inhibitors is normally 0.1 to 200ppm based on weight of a hydrocarbon mixture including conjugated diene. A method of using a polymerization inhibitor is not particularly limited and may be attained by simply bringing conjugated diene or a hydrocarbon mixture including conjugated diene contact the polymerization inhibitor.

### Operation and Effect of the Invention

In a method of separating and purifying conjugated diene by using separating and purifying apparatus according to the present invention, concentration of a polymerization inhibitor included in a bottom product taken out after a distillation is measured, and an supplying amount of the polymerization inhibitor to a distillation column is changed based thereon. Therefore, it is possible to supply a polymerization inhibitor in just proportion required for suppressing generation of popcorn polymer inside the apparatus, and generation of popcorn polymer can be efficiently and stably suppressed. As a result, costs of separating and purifying conjugated diene can be reduced comparing with that in the conventional techniques.

### BRIEF DESCRIPTION OF DRAWINGS

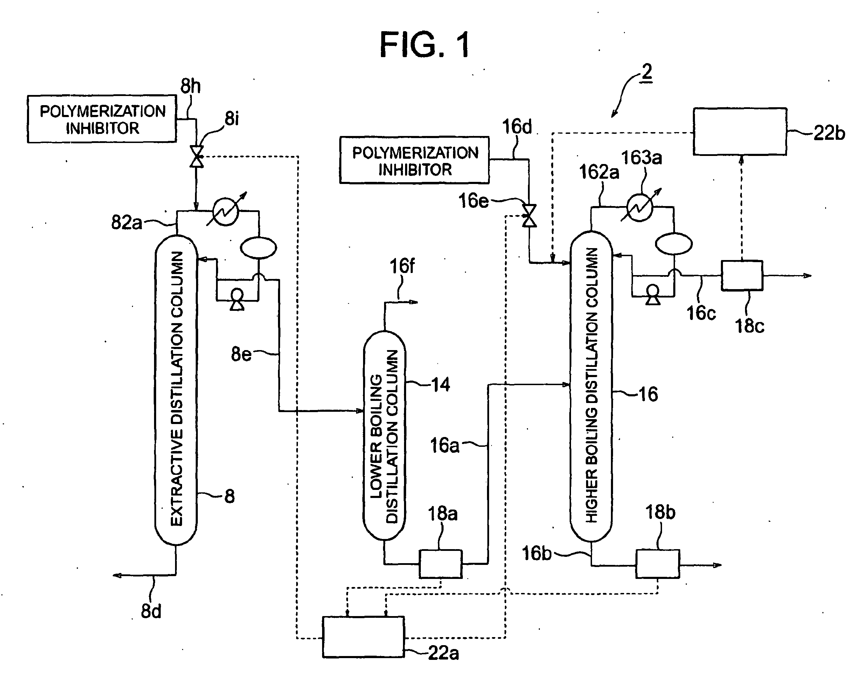

Figures 1 is a block diagram showing an example of a separation and purification system using a separation and purification apparatus used in the present invention.

| | |
|---|---|
| 2 | a separation and purification system |
| 8 | an extractive distillation column |
| 8d to 8e, 8h | a line |
| 82a | a reflux line |
| 8i | a supply amount adjustment valve |
| 14 | a lower boiling distillation column |
| 16 | a higher boiling distillation column |
| 16a to 16d, 16f | a line |
| 162a | a reflux line |
| 163a | a condenser |
| 16e | a supply amount adjustment valve |
| 22a, 22b | a control device (control means) |
| 18a to 18a | a liquid chromatograph or gas chromatograph analyzing device (measurement device) |

### BEST MODE FOR CARRYING OUT THE INVENTION

Below, an apparatus for separating and purifying conjugated diene used in the present invention and a method of separating and purifying conjugated diene according to the present invention will be explained in detail based on embodiments shown by the drawings. In the present embodiment, as an example of an apparatus for separating and purifying conjugated diene; an explanation will be made by taking an example of a separation and purification apparatus used for purifying a highly concentrated butadiene from C4 hydrocarbon fraction in which the concentration of butadiene is previously heightened by extractive distillation or so.

### Separation and Purification System 2

As shown in FIG. 1, a separation and purification system 2 according to the present embodiment comprises a lower boiling distillation column 14 and a higher boiling distillation column 16. The lower boiling distillation column 14 is a column for performing distillation of a C4 hydrocarbon fraction in which the concentration of butadiene is heightened and taking out a first distillation portion and a first bottom product, which is connected to the later explained higher boiling distillation column 16 by a line 16a. The line 16a is provided with a liquid chromatograph or gas chromatograph analyzing device (measurement device) 18a for measuring concentration of a polymerization inhibitor included in the first bottom product flowing there, and the measured concentration of the polymerization inhibitor is sent as a predetermined output signal to the control device (control means) 22a. According to the present embodiment, in a reflux line 82a provided near the top of the column body of extractive distillation column 8, a line 8h for supplying a polymerization inhibitor is connected.

On the line 8h, a supply amount adjustment valve 8i for adjusting a supply amount of the polymerization inhibitor is provided, and a supply amount of the polymerization inhibitor can be adjusted based on an output signal sent from a control device 22a.

The lower boiling distillation column 14 generally comprises a column body, a condenser for cooling vapor exhausted from the top of the column body and condensing to a liquid, a reflux drum for storing the distillation portion condensed to a liquid by the condenser, a reflux line for re-supply a part of the distillation portion stored in the reflux drum to near the top of the column body, and a re-boiler arranged at the bottom of the column body, but they are omitted in the drawing in the present embodiment. A form of the distillation column may be, for example, any of a plate column wherein inside the column body is divided by horizontal stage trays, so that contact of the liquid and vapor is made gradually, or a packed column filled with filler for efficient material movement between different phases, etc. Accuracy of distillation (ability of separating those having close boiling points) becomes higher as the number of stage trays becomes larger; however, when the number is too large, the cost is increased, so that it is selected in consideration of balance of the ability and the cost.

Note that the reference number "8d" in FIG. 1 is a line of taking out the extractive solvent from an extractive distillation column 8 at the bottom of the column body and the reference number "16f" is a line of taking out the first distillation portion from the reflux drum (not shown).

The higher boiling distillation column 16 is a column for distilling a first bottom product supplied through the line 16a and taking out a second distillation portion and a second bottom product. Near the upper stage of the higher boiling distillation column 16, a line 16d for supplying polymerization inhibitor is connected. Namely, according to the present embodiment, line 16d is not connected to reflux line 162a wherein the second distillation portion is taken out from the top of the column body of higher boiling distillation portion 16. Moreover, according to the present embodiment, connecting position of line 16d to the higher boiling distillation column 16, namely, supplying position of the polymerization inhibitor to the higher boiling distillation column 16 is movable in up-and-down motion in response to an output signal from the control device 22b. The line 16d is provided with a supply amount adjustment valve 16e for adjusting a supply amount of the polymerization inhibitor, and the supply amount of the polymerization inhibitor can be adjusted based on an output signal sent from the control device 22a.

Near the upper stage of the higher boiling distillation column 16, a line 16c for taking out the second distillation portion is connected. The line 16c is provided with a liquid chromatograph or a gas chromatograph analyzing device (measurement device) 18c for measuring concentration of a polymerization inhibitor included in the second distillation portion flowing there, and the measured concentration of the polymerization inhibitor is sent as a predetermined output signal to the control device (control means) 22b. Near the bottom stage of the higher boiling distillation column 16, a line 16b for taking out the second bottom product is connected. The line 16b is provided with a liquid chromatograph or a gas chromatograph analyzing device (measurement device) 18b for measuring concentration of a polymerization inhibitor included in the second bottom product flowing there, and the measured concentration of the polymerization inhibitor is sent as a predetermined output signal to the control device (control means) 22a. A form of the higher boiling distillation column 16, as mentioned above, may be a plate column or a packed column. When using a plate column, the number of stage trays is selected in consideration of balance of the ability and the cost as in the same way with the lower boiling distillation column 14 mentioned above.

### Effects

A separation and purification system 2 according to the present embodiment exhibits effects below.

The lower boiling distillation column 14 is supplied with a C4 hydrocarbon fraction in which the concentration of butadiene is heightened through a line 8e and a reflux line 82a arranged at near the top of the extractive distillation column 8. Moreover, the lower boiling distillation column 14 is supplied with the polymerization inhibitor through line 8e, which is from line 8h and mixed with the C4 hydrocarbon fraction in reflux line 82a. A position of supplying the C4 hydrocarbon fraction and the polymerization inhibitor to the lower boiling distillation column 14 is not particularly limited, but normally at an approximate middle stage. Further, the polymerization inhibitor is preferably supplied before a condenser in the reflux line 82a and mixed with the C4 hydrocarbon fraction. However, the polymerization inhibitor may be supplied from the other position, such as line 8e. Also, the line 8h may be directly connected to the lower boiling distillation column 14 and the polymerization inhibitor and C4 hydrocarbon fraction may be supplied separately. Along with the supply of the C4 hydrocarbon fraction and the polymerization inhibitor, distillation is performed by heating by the re-boiler (not shown) arranged at the bottom of the lower boiling distillation column 14. A pressure inside the lower boiling distillation column 14 is not particularly limited and is normally 4 to 6 atms, (405 to 608 kPa) and a temperature at the bottom is a boiling point under the pressure. From the top of the lower boiling distillation column 14, a distillation portion including much methyl acetylene is taken out, the distillation portion is condensed in the condenser (not shown), a part thereof is refluxed to be return to the top of the lower boiling distillation column 14, and the remaining portion is taken out as a first distillation portion through line 16f. On the other hand, from the bottom of the lower boiling distillation column 14, fraction including much high butadiene, cis-2-butene and pentene taken out as the first bottom product and supplied to the higher boiling distillation column 16 through the line 16a. And the higher boiling distillation column 16 is supplied with the polymerization inhibitor through line 16d.

A position of supplying the first bottom product to the higher boiling distillation column 16 is not particularly limited, but normally at an approximate middle stage and a position of supplying the polymerisation inhibitor to the higher boiling distillation column 16 is preferably at an upper stage.

The polymerization inhibitor used in here is not preferable to mix with a high purity butadiene, which may be a material of polybutadiene. When the supplying position of the polymerization inhibitor became higher, the effect of the polymerization inhibitor on the popcorn polymer also became high, however, the polymerization inhibitor was liable to mix with a high purity butadiene. When the supplying position of the polymerization inhibitor became lower, the polymerization inhibitor was difficult to mix with a high purity butadiene, however, the effect of the polymerization inhibitor on the popcorn polymer also became low.

Concretely, for instance, when the high boiling distillation column 16 is used as a plate column, the supplying position is preferably the first stage from the top. Along with the supply of the first bottom product and the polymerization inhibitor, distillation is performed by heating with the re-boiler (not shown) arranged at the bottom of the higher boiling distillation column 16. A pressure in the higher boiling distillation column 16 is not particularly limited, but normally is 4 to 6 atms (405 to 608 kPa), and a temperature at the bottom is a boiling X point under the pressure. From the bottom of the higher boiling distillation column 16, fraction including much high cis-2-butene and pentene are taken out as the second bottom product and recovered through line 16b.

On the other hand, a fraction including much highly concentrated butadiene is taken out from the top of the higher boiling distillation column 16, and the fraction is condensed in the condenser 163a. A part thereof is returned to the top of the higher boiling distillation column 16 through the reflux line 162a, and the remaining portion is taken out as a distillation portion through line 16c. Finally, the second distillation portion taken out from the line 16c is supplied as high purity butadiene to a material of polybutadiene, etc.

In the present embodiment, the line 16a is provided with a liquid chromatograph or a gas chromatograph analyzing device (measurement device) 18a for measuring concentration of a polymerization inhibitor included in the first bottom product. The measured concentration of the polymerization inhibitor is sent as an output signal (a concentration value) to the control device 22a, where the concentration value is compared with a reference range (concentration in a predetermined range). The concentration of the reference range depends on the kind of the polymerization inhibitor, however, with diethylhydroxylamine, preferably 1 to 20 ppm, more preferably 5 to 10 ppm. As a result, when the actually measured concentration value is judged to be over the upper limit value, the control device 22a sends to the valves 8i a signal to decrease a supply amount of the polymerization inhibitor to the lower boiling distillation column 14. Inversely, when the concentration of the polymerization inhibitor included in the first bottom product flowing in the line 16a is judged to be under the lower limit, the control device 22a sends to the valves 8i a signal to increase the supply amount of the polymerization inhibitor to the lower boiling distillation column 14. In the present embodiment, as explained above, on-off control is made to adjust the concentration of the polymerization inhibitor included in the first bottom product flowing in the line 16a to be in a reference range, but it may be controlled by other control algorithm, for example, proportional control, proportional integral control, proportional integral differentiation control, fuzzy control and adaptive control, etc.

In the present embodiment, the line 16b is provided with a liquid chromatograph or a gas chromatograph analyzing device (measurement device) 18b for measuring concentration of a polymerization inhibitor included in the second bottom product. The measured concentration of the polymerization inhibitor is sent as an output signal (a concentration value) to the control device 22a, where the concentration value is compared with a reference range (concentration in a predetermined range). The concentration of the reference range depends on the kind of the polymerization inhibitor, however, with diethylhydroxylamine, preferably 500 to 10000 ppm, more preferably 1500 to 8000 ppm. As a result, when the actually measured concentration value is judged to be over the upper limit value, the control device 22a sends to the valves 16e a signal to decrease a supply amount of the polymerization inhibitor to the higher boiling distillation column 16. After a predetermined time under these conditions, when the concentration of the polymerization inhibitor included in the second bottom product flowing in the line 16b is judged to be under the lower limit, the control device 22a sends to the valves 16e a signal to increase the supply amount of the polymerization inhibitor to the higher boiling distillation column 16.

In the present embodiment, as explained above, on-off control is made to adjust the concentration of the polymerization inhibitor included in the second bottom product flowing in the line 16b to be in a reference range, but it may be controlled by other abovementioned control algorithm. Further, in the present embodiment, the line 16c is provided with a liquid chromatograph or a gas chromatograph analyzing device (measurement device) 18c for measuring concentration of a polymerization inhibitor included in the second distillation portion. The measured concentration of the polymerization inhibitor is sent as an output signal (a concentration value) to the control device 22b, where the concentration value is compared with a reference value. The reference value is preferably 2ppm or less, more preferably 1ppm or less. As a result, when the actually measured concentration value is judged to be over the reference value, the control device 22b sends to the line 16d a signal to lower a supplying stage of the polymerization inhibitor to the higher boiling distillation column 16.

In the present embodiment, as explained above, on-off control is made to adjust the concentration of the polymerization inhibitor included in the second distillation portion flowing in the line 16c to be in a reference value, but it may be controlled by other abovementioned control algorithm.

Note that the measuring concentration of the polymerization inhibitor, as mentioned above, is not necessarily done on an on-line. The measuring may be performed on an off-line, namely, taking out a small amount of the distillation portion from each line, measuring concentration of the distillation portion at different place, and inputting the measurement result to the control devices 22a and 22b.

The separation and purification system 2 according to the present embodiment exhibits the effects described above, and it is possible to supply a polymerization inhibitor in just proportion required for suppressing generation of popcorn polymer, and generation of popcorn polymer can be efficiently and stably suppressed. As a result, high purity butadiene can be separated and purified at a lower cost comparing with the conventional cases.

Also, in the present embodiment, by measuring concentration of the polymerization inhibitor included in the first and the second bottom products flowing in line 16a and line 16b and the second distillation portion taken out from line 16c using a liquid chromatograph and a gas chromatograph analyzing devices 18a to 18c, measurement of the concentration of the polymerization inhibitor can be made easily and efficiently. Note that in the present embodiment, since an output signals are continuously sent from the analyzing devices 18a and 18b to the control device 22a and from the analyzing device 18c to the control device 22b respectively, the above operations are also continuously performed in the control devices 22a and 22b. However, a method in which the measurement interval is set to several hours to several days to send the signals to the control devices 22a and 22b (digital controlling) is more economical.

Embodiments of the present invention were explained above. The present invention may be naturally carried out in various embodiments within the scope of the present claims. For example, in the above embodiment, X butadiene is separated and purified from C4 hydrocarbon fraction, but isoprene may also be separated and purified from C5 hydrocarbon fraction.

## Claims

1. A method of separating and purifying conjugated diene, comprising the steps of:
obtaining a first bottom product by distilling a hydrocarbon mixture including 80% or more conjugated diene under an environment in the presence of a polymerization inhibitor in a lower boiling distillation column;
obtaining a second bottom product by distilling said first bottom product under an environment in the presence of a polymerization inhibitor in a higher boiling distillation column;
measuring each of concentrations of the polymerization inhibitors included in said first bottom product and second bottom product respectively; and
controlling the concentration of the polymerization inhibitor included in said first bottom product and/or second bottom product by changing a supply amount of the polymerization inhibitor to said lower boiling distillation column and/or higher boiling distillation column in accordance with said measured concentration of the polymerization inhibitor.

2. The method of separating and purifying conjugated diene as in claim 1 wherein the concentration of the conjugated diene is 90% or more.

3. The method of separating and purifying conjugated diene as in any one of the claims 1 or 2 wherein the polymerization inhibitor is di-lower alkylhydroxylamine.

4. The method of separating and purifying as in any one of the claims I to 3, wherein weight of the polymerization inhibitor is 0.1 to 200 ppm based on weight of a hydrocarbon mixture including conjugated diene.

5. The method of separating and purifying conjugated diene as in any one of the claims 1 to 4, wherein a hydrocarbon mixture including conjugated diene is a hydrocarbon mixture including conjugated diene of C4 or more.

6. Use of an apparatus for separating and purifying conjugated diene, wherein the apparatus comprises:
a lower boiling distillation column for obtaining a first bottom product by distilling a hydrocarbon mixture including 80% or more conjugated diene under an environment in the presence of a polymerization inhibitor;
a higher boiling distillation column for obtaining a second bottom product by distilling said first bottom product under an environment in the presence of the polymerization inhibitor,
a measurement means for measuring each of concentrations of the polymerization inhibitors included in said first bottom product and a second bottom product respectively,
a supply system for supplying the polymerization inhibitor newly to said lower boiling distillation column and/or higher boiling distillation column; and
a control means for controlling the concentration of the polymerization inhibitor included in said first bottom product and/or the second bottom product by changing a supply amount of the polymerization inhibitor to said lower boiling distillation column and/or higher boiling distillation column in accordance with said measured concentration of the polymerization inhibitor.

7. The use of the apparatus for separating and purifying conjugated diene as set forth in claim 6 wherein the measurement means for measuring said concentration is a liquid chromatograph analyzing apparatus or a gas chromatograph analyzing apparatus.

## Patentansprüche

1. Ein Verfahren zum Abtrennen und Reinigen von konjugiertem Dien, umfassend die Schritte:
Erhalten eines ersten Sumpfprodukts durch Destillieren eines Kohlenwasserstoffgemisches, das 80 % oder mehr konjugiertes Dien enthält, in einer Umgebung in Gegenwart eines Polymerisationsinhibitors in einer niedriger siedenden Destillationssäule;
Erhalten eines zweiten Sumpfprodukts durch Destillieren des ersten Sumpfprodukts in einer Umgebung in Gegenwart eines Polymerisationsinhibitors in einer höher siedenden Destillationssäule;
Messen der jeweiligen Konzentrationen der Polymerisationsinhibitoren, die im ersten Sumpfprodukt bzw. zweiten Sumpfprodukt enthalten sind; und
Regulieren der Konzentration des Polymerisationsinhibitors, der im ersten Sumpfprodukt und/oder zweiten Sumpfprodukt enthalten ist, durch Verändern einer Zugabemenge des Polymerisationsinhibitors zur niedriger siedenden Destillationssäule und/oder höher siedenden Destillationssäule gemäß der gemessenen Konzentration des Polymerisationsinhibitors.

2. Das Verfahren zum Abtrennen und Reinigen von konjugiertem Dien gemäß Anspruch 1, wobei die Konzentration des konjugierten Diens 90 % oder mehr beträgt.

3. Das Verfahren zum Abtrennen und Reinigen von konjugiertem Dien gemäß einem der Ansprüche 1 oder 2, wobei der Polymerisationsinhibitor ein di-Niederalkylhydroxylamin ist.

4. Das Verfahren zum Abtrennen und Reinigen von konjugiertem Dien gemäß einem der Ansprüche 1 bis 3, wobei das Gewicht des Polymerisationsinhibitors 0,1 bis 200 ppm, bezogen auf das Gewicht eines Kohlenwasserstoffgemisches, das konjugiertes Dien enthält, beträgt.

5. Das Verfahren zum Abtrennen und Reinigen von konjugiertem Dien gemäß einem der Ansprüche 1 bis 4, wobei ein Kohlenwasserstoffgemisch, das konjugiertes Dien enthält, ein Kohlenwasserstoffgemisch ist, das konjugiertes Dien von C4 oder mehr enthält.

6. Verwendung einer Vorrichtung zum Abtrennen und Reinigen von konjugiertem Dien, wobei die Vorrichtung umfasst:
eine niedriger siedende Destillationssäule zum Erhalten eines ersten Sumpfprodukts durch Destillieren eines Kohlenwasserstoffgemisches, das 80 % oder mehr eines konjugierten Diens enthält, in einer Umgebung in Gegenwart eines Polymerisationsinhibitors;
eine höher siedende Destillationssäule zum Erhalten eines zweiten Sumpfprodukts durch Destillieren des ersten Sumpfprodukts in einer Umgebung in Gegenwart des Polymerisationsinhibitors;
eine Messvorrichtung zum Messen der jeweiligen Konzentrationen der Polymerisationsinhibitoren, die im ersten Sumpfprodukt bzw. in einem zweiten Sumpfprodukt enthalten sind;
ein Zugabesystem zum frischen Zugeben des Polymerisationsinhibitors zur niedriger siedenden Destillationssäule und/oder höher siedenden Destillationssäule; und
eine Kontrollvorrichtung zum Regulieren der Konzentration des Polymerisationsinhibitors, der im ersten Sumpfprodukt und/oder im zweiten Sumpfprodukt enthalten ist, durch Verändern einer Zugabemenge des Polymerisationsinhibitors zur niedriger siedenden Destillationssäule und/oder höher siedenden Destillationssäule gemäß der gemessenen Konzentration des Polymerisationsinhibitors.

7. Die Verwendung der Vorrichtung zum Abtrennen und Reinigen von konjugiertem Dien wie in Anspruch 6 beansprucht, wobei die Messvorrichtung zum Messen der Konzentration ein Flüssigchromatographie-Analysegerät oder ein Gaschromatographie-Analysegerät ist.

## Revendications

1. Procédé de séparation et de purification d'un diène conjugué, comprenant les étapes consistant à :
obtenir un premier résidu par distillation d'un mélange d'hydrocarbures comprenant 80 % ou plus d'un diène conjugué dans un environnement en présence d'un inhibiteur de polymérisation dans une colonne de distillation de bas point d'ébullition ;
obtenir un second résidu par distillation dudit premier résidu dans un environnement en présence d'un inhibiteur de polymérisation dans une colonne de distillation de point d'ébullition élevé ;
mesurer chacune des concentrations des inhibiteurs de polymérisation inclus dans ledit premier résidu et ledit second résidu respectivement ; et
ajuster la concentration de l'inhibiteur de polymérisation inclus dans ledit premier résidu et/ou ledit second résidu en modifiant une quantité d'alimentation de l'inhibiteur de polymérisation à ladite colonne de distillation de bas point d'ébullition et/ou ladite colonne de distillation de point d'ébullition élevé, conformément à ladite concentration d'inhibiteur de polymérisation mesurée.

2. Procédé de séparation et de purification d'un diène conjugué selon la revendication 1, dans lequel la concentration du diène conjugué est de 90 % ou plus.

3. Procédé de séparation et de purification d'un diène conjugué selon la revendication 1 ou 2, dans lequel l'inhibiteur de polymérisation est une di(alkyle inférieur)hydroxylamine.

4. Procédé de séparation et de purification d'un diène conjugué selon l'une quelconque des revendications 1 à 3, dans lequel le poids de l'inhibiteur de polymérisation est de 0,1 à 200 ppm sur la base du poids d'un mélange d'hydrocarbures comprenant un diène conjugué.

5. Procédé de séparation et de purification d'un diène conjugué selon l'une quelconque des revendications 1 à 4, dans lequel un mélange d'hydrocarbures comprenant un diène conjugué est un mélange d'hydrocarbures comprenant un diène conjugué en C4 ou plus.

6. Utilisation d'un appareil pour la séparation et la purification d'un diène conjugué, l'appareil comprenant :
une colonne de distillation de bas point d'ébullition pour obtenir un premier résidu par distillation d'un mélange d'hydrocarbures comprenant 80 % ou plus d'un diène conjugué dans un environnement en présence d'un inhibiteur de polymérisation ;
une colonne de distillation de point d'ébullition élevé pour obtenir un second résidu par distillation dudit premier résidu dans un environnement en présence de l'inhibiteur de polymérisation ;
un moyen de mesure pour mesurer chacune des concentrations des inhibiteurs de polymérisation inclus dans ledit premier résidu et ledit second résidu respectivement ;
un système d'alimentation pour alimenter le nouvel inhibiteur de polymérisation à ladite colonne de distillation de bas point d'ébullition et/ou ladite colonne de distillation de point d'ébullition élevé ; et
un moyen d'ajustement pour ajuster la concentration de l'inhibiteur de polymérisation inclus dans ledit premier résidu et/ou ledit second résidu en modifiant une quantité d'alimentation de l'inhibiteur de polymérisation à ladite colonne de distillation de bas point d'ébullition et/ou ladite colonne de distillation de point d'ébullition élevé, conformément à ladite concentration d'inhibiteur de polymérisation mesurée.

7. Utilisation d'un appareil pour la séparation et la purification d'un diène conjugué selon la revendication 6, dans laquelle le moyen de mesure pour mesurer ladite concentration est un appareil d'analyse par chromatographie liquide ou un appareil d'analyse par chromatographie gazeuse.
